# EUROPEAN PATENT SPECIFICATION

(11) **EP 2 532 252 B1**
(45) Date of publication and mention of the grant of the patent: **09.09.2015**
(21) Application number: 12169839.3
(22) Date of filing: 29.05.2012
(51) Int. Cl.: A23L 1/30, C02F 1/04, C02F 1/44, C02F 3/34, A61K 36/63, A23L 1/305, A23L 2/52, A23L 2/66, A61P 17/00

(54) **Beverage to ameliorate skin condition**
Getränk zur Verbesserung des Hautzustands
Boisson pour améliorer l' état de la peau

(30) Priority: 06.06.2011 IT TO20110488
(43) Date of publication of application: 12.12.2012
(73) Proprietor: Minerva Research Labs Ltd, London W1J 7BU (GB)
(72) Inventor: Sanguinetti, Catone Tony, London, W1J 7BU (GB); Kume, Shoko, London, W1J 7BU (GB); Aung, Thein, London, W1J 7BU (GB)
(74) Representative: Freyria Fava, Cristina

(56) References cited:
- EP-A2- 1 075 836
- WO-A1-2008/079898
- WO-A2-2011/011604
- JP-A- 2008 150 326
- JP-A- 2009 142 180
- US-A1- 2009 269 419

## Description

### Field of the invention

This disclosure concerns a beverage particularly suitable for improving skin condition.

### Background of the invention

The main functions of the skin are to provide to the body a mechanical protection and a chemical barrier able to restrain the foreign substance penetration, to prevent water or endogenous fluid loss and to keep a constant temperature. In addition, the skin also protect against ultraviolet irradiation and pathogen invasion.

Skin is subject to deterioration through dermatological disorders, environmental condition (wind, air conditioning, heating) or through the normal ageing process which may be accelerated by exposure of the skin to sun (photoageing).

In recent years the demand for cosmetic compositions and cosmetic methods for improving the appearance and condition of the skin has grown enormously.

The cosmetic and dermatological industry aims to prevent the skin dehydration, the appearance of dry skin or of fine lines and wrinkles by topical application of creams and lotions containing ingredients able to improve the skin quality.

This effect is, however, only local and the benefit is only provided to the part of the skin where the lotion or the cream are applied, that is on the outer skin layer, namely the epidermis.

In contrast, the dermis - the layer localized between the epidermis and the subcutaneous tissues - which confers elasticity and firmness to the skin is not easily achievable by local application.

The use of ingredients that can improve the skin condition is beneficial especially when they are orally administered thanks to the blood and interstitial fluids delivery system that distribute them to the whole body and to the entire thickness of the skin. A health drink supplying sugar chain nutritious elements able, for example, to increase the moisture holding ability of the skin is known from JP-A-2008150326.

### Summary of the invention

The object of this disclosure is to provide a beverage for improving skin quality, particularly by improving hydration, firmness and elasticity of both epidermis and dermis through the synergistic effects exerted by its ingredients and by means of the administration route.

According to the invention, the above object is achieved thanks to the subject matter recalled specifically in the ensuing claims, which are understood as forming an integral part of this disclosure.

An embodiment of the present disclosure provides a beverage comprising collagen, hyaluronic acid, borage oil emulsion and a glucosamine derivative selected between N-acetylglucosamine and glucosamine hydrochloride.

In another embodiment, the beverage also comprises at least one between black pepper extract and curcumin emulsion.

In a further embodiment the beverage herein described also includes vitamins, additives, carbohydrate sources, amino acids and flavouring substances.

### Detailed description of the invention

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in convection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The headings provided herein are for convenience only and do not interpret the scope or meaning of the embodiments.

An embodiment of the present disclosure provides a beverage comprising collagen, hyaluronic acid, borage oil emulsion and a glucosamine derivative selected between N-acetylglucosamine and glucosamine hydrochloride.

The beverage herein described is able to reduce skin dryness and to improve firmness and elasticity of the skin, skin softness and glow, mainly acting on the dermis. In addition, it also exerts an anti-ageing effect.

The beverage herein described comprises collagen in an amount between 1 and 20 g/100 ml, preferably in an amount between 2 and 15 g/100 ml, more preferably in an amount between 3 and 10 g/100 ml. The collagen used in the.beverage is preferably hydrolyzed collagen, more preferably deriving from a fish source.

The hyaluronic acid is present in an amount between 0.5 and 100 mg/100 ml, preferably in an amount between 1 and 75 mg/100 ml, more preferably in an amount between 2 and 40 mg/100 ml.

The borage oil emulsion is present in the beverage in an amount between 1 and 500 mg/100 ml, preferably in an amount between 5 and 350 mg/100 ml, more preferably in an amount between 10 and 200 mg/100 ml.

The borage oil is insoluble in water. The emulsion comprises glycerol, lecithin and borage oil which is preferably present in an amount of 20% by weight with respect to the total weight of the emulsion. The borage oil emulsion has hydrophilic properties thus is able to increase its solubility in a water environment along with the other active ingredients in the beverage.

The glucosamine derivative, N-acetylglucosamine or glucosamine hydrochloride, is present in an amount between 1 and 50 mg/100 ml, preferably in an amount between 2 and 20 mg/100 ml, more preferably in an amount between 3 and 10 mg/100 ml.

Collagen and hyaluronic acid are vital, naturally present components of healthy skin tissues. Collagen is the main structural protein of the skin and confers tensile strength and elasticity.

Hyaluronic acid is able to retain and hold moisture playing an important role in maintaining hydration and a youthful look of the skin.

It has been shown that both collagen and hyaluronic acid decline with age leading to a loss of the skin elasticity and to the formation of fine lines and wrinkles.

N-acetylglucosamine is another essential component of the skin tissues needed for the body production of hyaluronic acid. Glucosamine hydrochloride has been proven to help retain moisture and reduce the appearance of fine lines and wrinkles.

Borage oil, derived from the seeds of the *Borago officinalis* (borage), is one of the richest sources of gamma-linolenic acid (GLA) containing omega-6 essential fatty acids (EFAs).

Borage oil is an important ingredient used to improve skin hydration because it plays a vital role in preserving the natural barrier of the skin against water-loss and in the maintenance of the fluidity and stability of the dermal cells. By preventing water-loss, GLA containing omega-6 fatty acids contributes to the proper and healthy functioning of the cellular membranes and can actually prevent dryness of the skin.

Due to an unexpected synergy among the effects exerted by its main ingredients, i.e. the collagen, the hyaluronic acid, the glucosamine derivative selected between N-acetylglucosamine and glucosamine hydrochloride and the borage oil, and the oral administration route, the beverage herein described leads to an improvement of skin hydration, firmness and elasticity, mainly acting on the dermis.

In another embodiment, the beverage also comprises at least one between a black pepper extract and a curcumin emulsion.

The black pepper extract, known to enhance bioavailability of several substances by increasing their gastrointestinal absorption, can be present in an amount between 0.2 and 5 mg/100 ml, preferably between 0.8 and 4 mg/100 ml.

The curcumin emulsion, preferably containing curcumin in an amount of 20% by weight, can be present in the beverage in an amount between 15 and 200 mg/100 ml, preferably 30 and 150 mg/100 ml, more preferably 50 and 100 mg/100 ml.

Curcumin is the principal curcuminoid of the popular Indian spice turmeric, a herb used for centuries in Chinese and Indian Ayurvedic medicine. It is the active ingredient form of turmeric and exhibits anti-oxidant, anti-bacterial and anti-inflammatory properties.

The present Inventors found that by adding the black pepper extract and/or the curcumin emulsion to the beverage, a further potentiating effect in ameliorating skin condition is obtained.

In a further embodiment of the present disclosure the beverage herein described also includes at least one among: vitamins, additives, carbohydrate sources, amino acids and flavouring substances.

The vitamins useful in the beverage are any vitamin known to have a health benefit to consumers.

Preferably, the vitamin is selected from the group consisting of vitamin A, vitamin B₁, vitamin B₂, vitamin B₆, vitamin B₁₂, vitamin C, vitamin D, vitamin E, and other water soluble vitamins.

Vitamin B6 brings energy and vitality. Vitamin C contributes to normal collagen formation and the normal function of bones, teeth, cartilage, gums, skin and blood vessels. Vitamin E provides protection of body cells from oxidative damage by acting as a free radical scavenger.

The additive is preferably selected among citric acid anhydrous, phosphoric acid, lactic acid, tartaric acid, DL-malic acid, sucralose, potassium sorbate and sodium benzoate.

The carbohydrate source is selected from glucose-fructose syrup, sucrose and maltodextrin.

The amino acid that may be contained in the beverage is selected among taurine, DL-Alanine, DL-Methionine, glycine, L-arginine, L-ornithine.

Flavouring substances may be selected among orange essential oil and grapefruit essential oil.

Lactones, esters, aliphatic higher alcohols, ketones, aromatic aldehydes, aromatic alcohols, thioethers, fatty acids, propylene glycol, ethanol, glycerol (glycerine) were used to formulate the flavor base.

Table 1 reports the content range of the ingredients that can be included in the water based beverage as described above.

**Table 1**

| **Ingredients** | **Content range/100 ml** |
|---|---|
| Collagen (hydrolysed fish collagen) | 1-20 g |
| Hyaluronic acid | 0.5-100 mg |
| Borage oil emulsion (20%) | 1-500 mg |
| N-acetylglucosamine | 1-50 mg |
| Black pepper extract | 0.2-5 mg |
| Curcumin emulsion (20%) | 15-200 mg |
| Vitamins | 0-800 mg |
| Additives | 0-2000 mg |
| Carbohydrate sources | 0-6000 mg |
| Amino acids | 0-200 mg |
| Flavouring substances | 0-400 mg |

In the following two illustrating, non limiting examples of beverages according to the instant description are provided.

### Example 1.

The beverage comprising the ingredients reported in Table 2 below has been prepared first by mixing and dissolving at a temperature of 60±5°C in a mixing tank water, hyaluronic acid, taurine, hydrolysed fish collagen, N-acetylglucosamine, citric acid anhydrous, DL-malic acid, black pepper extract, sucralose, pyridoxine hydrochloride, ascorbic acid and potassium sorbate. Then water, borage oil emulsion (20%), d-α-tocopherol and curcumin emulsion (20%) are added into the mixing tank lowering the temperature at 20±5°C. Finally, the flavouring substances and the glucose-fructose syrup are also included. After mixing all the ingredients, water is added in order to adjust the volume to 100 ml.

**Table 2**

| **Ingredients** | **Content/100 ml** |
|---|---|
| Collagen (hydrolysed fish collagen) | 5 g |
| Hyaluronic acid | 20 mg |
| Borage oil emulsion (20%) | 50 mg |
| N-acetylglucosamine | 5 mg |
| Vitamin B₆ (Pyridoxine Hydrochloride) | 1.65 mg |
| Vitamin C (Ascorbic acid) | 240 mg |
| Vitamin E (d-α-tocopherol 4%) | 300 mg |
| DL-Malic acid | 100 mg |
| Sucralose | 2 mg |
| Curcumin emulsion (20%) | 50 mg |
| Black pepper extract | 2 mg |
| Citric acid anhydrous | 700 mg |
| Glucose-fructose syrup | 3 g |
| Taurine | 100 mg |
| Potassium sorbate | 200 mg |
| Flavouring substances | 121 mg |
| Water | 92 g |

### Example 2.

The beverage (in a different volume package) comprising the ingredients reported in Table 3 below has been prepared first by mixing and dissolving at a temperature of 60±5°C in a mixing tank water, hyaluronic acid, hydrolysed fish collagen, N-acetylglucosamine, citric acid anhydrous, DL-malic acid, black pepper extract, sucralose, pyridoxine hydrochloride, ascorbic acid and potassium sorbate. Then water, borage oil emulsion (20%) and d-α-tocopherol are added into the mixing tank lowering the temperature at 20±5°C. Finally, the flavouring substances and the glucose-fructose syrup are also included. After mixing all the ingredients, water is added in order to adjust the volume to 50 ml.

**Table 3**

| **Ingredients** | **Content/50 ml** |
|---|---|
| Collagen (hydrolysed fish collagen) | 5 g |
| Hyaluronic acid | 20 mg |
| Borage oil emulsion (20%) | 50 mg |
| N-acetylglucosamine | 5 mg |
| Vitamin B₆ (Pyridoxine hydrochloride) | 1.65 mg |
| Vitamin C (Ascorbic acid) | 240 mg |
| Vitamin E (d-α-tocopherol 4%) | 150 mg |
| DL-Malic acid | 100 mg |
| Sucralose | 2 mg |
| Black pepper extract | 1.5 mg |
| Citric acid anhydrous | 400 mg |
| Glucose-fructose syrup | 2 g |
| Potassium sorbate | 100 mg |
| Flavouring substances | 108.7 mg |
| Water | 43 g |

The beverages realized as described above administered to humans had ameliorating effects on the overall state of the skin.

In particular, the beverage intake reduced skin dryness, improved firmness and elasticity and exerted a purported anti-ageing effect.

The realization of the beverage is not limited to these examples but can have variants, which are not exceeding the limits of the underwritten claims.

Naturally, while the principle of the invention remains the same, the details of construction and the embodiments may widely vary with respect to what has been described and illustrated purely by way of example, without departing from the scope of the present invention.

## Claims

1. A beverage comprising collagen, hyaluronic acid, borage oil emulsion and a glucosamine derivative, wherein said glucosamine derivative is selected between N-acetylglucosamine and glucosamine hydrochloride.

2. A beverage according to claim 1, wherein said collagen is present in an amount between 1 and 20 g/100 ml, preferably 2 and 15 g/100 ml, more preferably 3 and 10 g/100 ml.

3. A beverage according to claim 1, wherein said hyaluronic acid is present in an amount between 0.5 and 100 mg/100 ml, preferably between 1 and 75 mg/100 ml, more preferably between 2 and 40 mg/100 ml.

4. A beverage according to claim 1, wherein said borage oil emulsion, preferably containing borage oil in an amount of 20% by weight, is present in an amount between 1 and 500 mg/100 ml, preferably 5 and 350 mg/100 ml, more preferably 10 and 200 mg/100 ml.

5. A beverage according to claim 1, wherein said glucosamine derivative is present in an amount between 1 and 50 mg/100 ml, preferably between 2 and 20 mg/100 ml, more preferably between 3 and 10 mg/100 ml.

6. A beverage according to claim 1 further comprising at least one between a black pepper extract and a curcumin emulsion.

7. A beverage according to any one of the preceding claims, wherein said beverage further comprises at least one among: a vitamin, an additive, a carbohydrate source, a nutritional supplement and a flavoring substance.

8. A beverage according to claim 7, wherein said vitamin is selected among: vitamin A, vitamin B₁, vitamin B₂, vitamin B₆, vitamin B₁₂, vitamin C, vitamin D, vitamin E.

9. A beverage according to claim 7 or claim 8, wherein said additive is selected among: citric acid anhydrous, phosphoric acid, lactic acid, tartaric acid, DL-malic acid, sucralose, potassium sorbate and sodium benzoate.

10. A beverage according to any one or claims 7 to 9, wherein said carbohydrate source is selected among: glucose-fructose syrup, sucrose, maltodextrin.

11. A beverage according to any one of claims 7 to 10, wherein said aminoacid is selected among: taurine, DL-Alanine, DL-Methionine, glycine, L-arginine, L-ornithine.

12. A beverage according to any one of claims 7 to 11, wherein said flavouring substance is selected among: orange essential oil, grapefruit essential oil.

13. Use of collagen, hyaluronic acid, borage oil emulsion and a glucosamine derivative selected between N-acetylglucosamine and glucosamine hydrochloride for improving skin condition, preferably reducing skin dryness and improving skin firmness, elasticity, softness and glow.

## Patentansprüche

1. Getränk umfassend Collagen, Hyaluronsäure, eine Borretschöl-Emulsion und ein Glucosaminderivat, wobei das Glucosaminderivat aus N-Acetylglucosamin und Glucosamin-Hydrochlorid ausgewählt ist.

2. Getränk nach Anspruch 1, wobei das Collagen in einer Menge zwischen 1 und 20 g/100 ml, vorzugsweise 2 und 15 g/100 ml, mehr bevorzugt 3 und 10 g/100 ml vorhanden ist.

3. Getränk nach Anspruch 1, wobei die Hyaluronsäure in einer Menge zwischen 0,5 und 100 mg/100 ml, vorzugsweise zwischen 1 und 75 mg/100 ml, mehr bevorzugt zwischen 2 und 40 mg/100 ml vorhanden ist

4. Getränk nach Anspruch 1, wobei die Borretschöl-Emulsion, die vorzugsweise Borretschöl in einer Menge von 20 Gew.-% enthält, in einer Menge zwischen 1 und 500 mg/100 ml, vorzugsweise 5 und 350 mg/100 ml, mehr bevorzugt 10 und 200 mg/100 ml vorhanden ist.

5. Getränk nach Anspruch 1, wobei das Glucosaminderivat in einer Menge zwischen 1 und 50 mg/100 ml, vorzugsweise zwischen 2 und 20 mg/100 ml, mehr bevorzugt zwischen 3 und 10 mg/100 ml vorhanden ist.

6. Getränk nach Anspruch 1, außerdem umfassend wenigstens eines von einem Extrakt von schwarzem Pfeffer und einer Curcumin-Emulsion.

7. Getränk nach einem der vorangehenden Ansprüche, wobei das Getränk außerdem wenigstens eines von einem Vitamin, einem Zusatz, einer Kohlenhydratquelle, einem Nahrungsergänzungsmittel und einem Aromastoff umfasst.

8. Getränk nach Anspruch 7, wobei das Vitamin ausgewählt ist aus: Vitamin A, Vitamin B₁, Vitamin B₂, Vitamin B₆, Vitamin B₁₂, Vitamin C, Vitamin D, Vitamin E.

9. Getränk nach Anspruch 7 oder Anspruch 8, wobei der Zusatz ausgewählt ist aus: wasserfreier Citronensäure, Phosphorsäure, Milchsäure, Weinsäure, DL-Äpfelsäure, Sucralose, Kaliumsorbat und Natriumbenzoat.

10. Getränk nach einem der Ansprüche 7 bis 9, wobei die Kohlenhydratquelle ausgewählt ist aus: Glucose-Fructose-Sirup, Sucrose, Maltodextrin.

11. Getränk nach einem der Ansprüche 7 bis 10, wobei die Aminosäure ausgewählt ist aus: Taurin, DL-Alanin, DL-Methionin, Glycin, L-Arginin, L-Ornithin.

12. Getränk nach einem der Ansprüche 7 bis 11, wobei der Aromastoff ausgewählt ist aus: ätherischem Orangenöl und ätherischem Grapefruitöl.

13. Verwendung von Collagen, Hyaluronsäure, einer Borretschöl-Emulsion und einem Glucosaminderivat, ausgewählt aus N-Acetylglucosamin und Glucosamin-Hydrochlorid, zum Verbessern des Hautzustandes, vorzugsweise zum Verringern der Hauttrockenheit und zum Verbessern der Festigkeit, Elastizität, Weichheit und des Leuchtens der Haut.

## Revendications

1. Boisson comprenant du collagène, de l'acide hyaluronique, une émulsion d'huile de bourrache et un dérivé de glucosamine, où ledit dérivé de glucosamine est choisi entre la N-acétylglucosamine et le chlorhydrate de glucosamine.

2. Boisson selon la revendication 1, dans laquelle ledit collagène est présent en une quantité comprise entre 1 et 20 g/100 ml, de préférence entre 2 et 15 g/100 ml, plus préférablement entre 3 et 10 g/100 ml.

3. Boisson selon la revendication 1, dans laquelle ledit acide hyaluronique est présent en une quantité comprise entre 0,5 et 100 mg/100 ml, de préférence entre 1 et 75 mg/100 ml, plus préférablement entre 2 et 40 mg/100 ml.

4. Boisson selon la revendication 1, dans laquelle ladite émulsion d'huile de bourrache, contenant de préférence de l'huile de bourrache en une quantité de 20% en poids, est présente en une quantité comprise entre 1 et 500 mg/100 ml, de préférence entre 5 et 350 mg/100 ml, plus préférablement entre 10 et 200 mg/100 ml.

5. Boisson selon la revendication 1, dans laquelle ledit dérivé de glucosamine est présent en une quantité comprise entre 1 et 50 mg/100 ml, de préférence entre 2 et 20 mg/100 ml, plus préférablement entre 3 et 10 mg/100 ml.

6. Boisson selon la revendication 1, comprenant en outre au moins l'un(e) entre un extrait de poivre noir et une émulsion de curcumine.

7. Boisson selon l'une quelconque des revendications précédentes, dans laquelle ladite boisson comprend en outre au moins l'un(e) parmi : une vitamine, un additif, une source de glucides, un complément nutritionnel et une substance aromatisante.

8. Boisson selon la revendication 7, dans laquelle ladite vitamine est choisie parmi : la vitamine A, la vitamine B₁, la vitamine B₂, la vitamine B₆, la vitamine B₁₂, la vitamine C, la vitamine D, la vitamine E.

9. Boisson selon la revendication 7 ou 8, dans laquelle ledit additif est choisi parmi : l'acide citrique anhydre, l'acide phosphorique, l'acide lactique, l'acide tartrique, l'acide DL-malique, le sucralose, le sorbate de potassium et le benzoate de sodium.

10. Boisson selon l'une quelconque des revendications 7 à 9, dans laquelle ladite source de glucides est choisie parmi : le sirop de glucose-fructose, le saccharose, la maltodextrine.

11. Boisson selon l'une quelconque des revendications 7 à 10, dans laquelle ledit aminoacide est choisi parmi : la taurine, la DL-Alanine, la DL-Méthionine, la glycine, la L-arginine, la L-ornithine.

12. Boisson selon l'une quelconque des revendications 7 à 11, dans laquelle ladite substance aromatisante est choisie parmi : l'huile essentielle d'orange, l'huile essentielle de pamplemousse.

13. Utilisation de collagène, d'acide hyaluronique, d'émulsion d'huile de bourrache et d'un dérivé de glucosamine choisi entre la N-acétylglucosamine et le chlorhydrate de glucosamine pour améliorer l'état de la peau, de préférence pour réduire la sécheresse de la peau et pour améliorer la fermeté, l'élasticité, la douceur et l'éclat de la peau.
